(19) Europäisches Patentamt
European Patent Office
Office européen des brevets

(11) Veröffentlichungsnummer: **0 200 068**
**A2**

(12) # EUROPÄISCHE PATENTANMELDUNG

(21) Anmeldenummer: **86105089.6**

(22) Anmeldetag: **14.04.86**

(51) Int. Cl.⁴: **A 61 K 9/50**
**A 61 K 31/44**

(30) Priorität: **27.04.85 DE 3515335**

(43) Veröffentlichungstag der Anmeldung:
**05.11.86 Patentblatt 86/45**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Schlossmann, Klaus, Dr.**
**In der Beek 116**
**D-5600 Wuppertal 1(DE)**

(72) Erfinder: **Zembrod, Alfred, Dr.**
**Nittumer Weg 60**
**D-5060 Bergisch-Gladbach 2(DE)**

(72) Erfinder: **Kazda, Stanislav, Dr.**
**Gellertweg 18**
**D-5600 Wuppertal 1(DE)**

(72) Erfinder: **Serno, Peter, Dr.**
**An der Ruthen 3**
**D-5000 Koeln 80(DE)**

(72) Erfinder: **Klinksiek, Bernd, Dl.**
**Obervolbach 10**
**D-5060 Bergisch-Gladbach 4(DE)**

(54) **Arzneizubereitung enthaltend Dihydropyridine und Verfahren zu ihrer Herstellung.**

(57) Parenteral applizierbare Arzneizubereitung, welche Liposomen mit einem mittleren Durchmesser von 20 - 1000 nm enthält, wobei die Liposomenmembran folgende Bestandteile aufweist:

a) 1 Gewichtsteil eines Dihydropyridins
b) 5-500 Gewichtsteile Lipide

und wobei die Liposomen in einem wäßrigen Medium verteilt sind, welche einen pH-Wert von 6,5 - 8,0 aufweist, sowie ein Verfahren zu ihrer Herstellung, das dadurch gekennzeichnet ist, daß man 1 Gewichtsteil des Dihydropyridins und 5-500 Gewichtsteile Lipid in organischen Lösungsmitteln löst, anschließend das Lösungsmittel entfernt und den Rückstand nach Zugabe eines wäßrigen Mediums mit einem pH-Wert von 6,5 - 8 bei Temperaturen zwischen 20 und 80°C dispergiert.

EP 0 200 068 A2

BAYER AKTIENGESELLSCHAFT 5090 Leverkusen, Bayerwerk

Konzernverwaltung RP
Patentabteilung KS/Kü-c

Arzneizubereitung enthaltend Dihydropyridine und Verfahren zu ihrer Herstellung

Die Erfindung betrifft eine parenteral applizierbare Arzneizubereitung enthaltend Dihydropyridine in der Matrix von Liposomen sowie ein Verfahren zu ihrer Herstellung.

Es ist bekannt, daß Dihydropyridine sehr starke kreislaufbeeinflussende Wirkungen besitzen und sich somit beispielsweise zur Bekämpfung des Bluthochdruckes, von Herzerkrankungen und ischämischen Gehirnerkrankungen eignen (vgl. britisches Patent 1 173 862).

Aufgrund ihrer Lichtempfindlichkeit und ihrer schweren Löslichkeit treten bei der galenischen Zubereitung von Arzneizubereitungen auf Basis der Dihydropyridine eine Reihe von Schwierigkeiten auf.

Alle bisherigen Versuche, die schlechte Löslichkeit z.B. von Nimodipin durch bestimmte Maßnahmen zu kompensieren und gleichzeitig eine gute Bioverfügbarkeit zu gewährleisten, besitzen eine Reihe von Nachteilen. Der Einsatz von oberflächenaktiven Substanzen, Lösungsvermittlern und bestimmten Trägerstoffen, die eine besondere Oberfläche haben, führt häufig zu Verabreichungsformen bei denen die Präparate unerwünscht groß sind. Zur Erleichterung des Schluckens werden solche Tabletten oder Kapseln häufig in spezifische Formen wie z.B. Elypsoide oder Längsformen überführt, was jedoch bei Präparaten mit einem Gewicht über 400 mg auch nicht mehr zu befriedigenden Ergebnissen führt. Auch das häufigere Einnehmen von kleineren Präparaten stellt keine befriedigende Lösung dar.

Zur Bekämpfung einiger Krankheiten z.B. der ischämischen Gehirnerkrankungen ist es wünschenswert, die Dihydropyridine in einer Lösung parenteral (intravenös oder intraarteriell) zu applizieren.

Da, wie geschildert, die Dihydropyridine in Wasser schwer löslich sind, mußte bisher für die parenterale Applikation mit Hilfe von organischen Lösungsmitteln eine injizierbare Zubereitung formuliert werden.

Wie bekannt, ist die Verwendung von organischen Lösungsmitteln vom klinisch-therapeutischen Standpunkt gesehen nicht unbedenklich. So können beispielsweise Gewebeschäden und lokale Reizeffekte an den Gefäßen auftreten.

Derartige Nebeneffekte bei der parenteralen Applikation von Dihydropyridinen schränken die Verwendung organischer Lösungsmittel ein.

Es wurde nunmehr gefunden, daß die Dihydropyridinverbindungen sich ohne Zusatz von organischen Lösungsmitteln applizieren lassen, falls sie in der Matrix von Liposomen eingebaut sind.

Die Erfindung betrifft somit eine parenteral applizierbare Arzneizubereitung, welche Liposomen mit einem mittleren Durchmesser von 20-1000 nm, bevorzugt 50-200 nm, enthält, wobei die Liposomenmembran folgende Bestandteile aufweist:

a) 1 Gewichtsteil eines Dihydropyridins;

b) 5-500 Gewichtsteile, bevorzugt 20-200 Gew.-Teile, Lipide

und wobei diese Liposomen in einem wäßrigen Medium verteilt sind, welches einen pH-Wert von 3,0 bis 8,5 aufweist.

Die Dihydropyridine sind in der Membran der Liposomen verteilt.

Unter Liposomen versteht man artifiziell hergestellte Vesikel, deren Membranmaterial hauptsächlich aus natürlichen Membrankomponenten wie Phospholipiden besteht. Ist eine Verbindung lipidlöslich wie die Dihydropyridinderivate, läßt sie sich in die Lipidmembran einbauen im Gegensatz zu wasserlöslichen Stoffen, die bei der Präparation der Liposomen im wäßrigen Innenvolumen der Vesikel eingeschlossen werden.

Als Dihydropyridine seien bevorzugt aufgeführt Verbindungen der Formel I

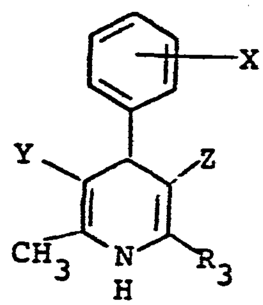

(I)

in der

Y die Gruppe $-CO_2R_1$,

$R_1$ $C_1$-$C_4$-Alkyl, gegebenenfalls substituiert durch $C_1$-$C_3$-Alkoxy,

Z die Gruppe $-COOR_2$,

$R_2$ $C_1$-$C_{10}$-Alkyl, gegebenenfalls substituiert durch $C_1$-$C_3$-Alkoxy, Trifluormethyl, N-Methyl-N-benzylamino, Benzyl,

$R_3$ $C_1$-$C_4$-Alkyl, Cyano, Hydroxymethyl und

X 2- bzw. 3-Nitro, 2,3-Dichlor, 2,3-Ringglied bestehend aus =N-O-N=, 2 bzw. 3-Trifluormethyl

bedeuten.

Besonders bevorzugt seien die Verbindungen der folgenden Tabelle genannt:

<u>Tabelle</u>

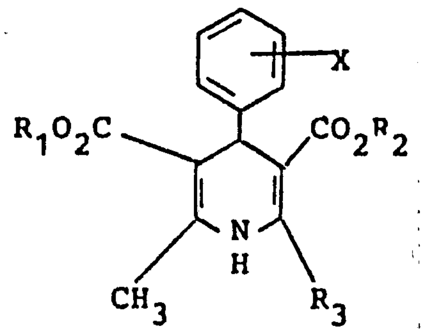

| Nr. | X | $R^1$ | $R^2$ | $R^3$ | Generic |
|---|---|---|---|---|---|
| 1 | $2-NO_2$ | $CH_3$ | $CH_3$ | $CH_3$ | Nifedipin |
| 2 | $3-NO_2$ | $nPrOCH_2CH_2$ | $nPrOCH_2CH_2$ | $CH_3$ | Niludipin |
| 3 | $3-NO_2$ | $C_2H_5$ | $CH_3$ | $CH_3$ | Nitrendipin |
| 4 | $2-NO_2$ | $CH_3$ | $(CH_3)_2CHCH_2$ | $CH_3$ | Nisoldipin |
| 5 | $3-NO_2$ | $CH(CH_3)_2$ | $(CH_2)_2-O-CH_3$ | $CH_3$ | Nimodipin |
| 6 | $3-NO_2$ | $C_2H_5$ | $C_{10}H_{21}(n)$ | $CH_3$ | |
| 7 | 2-Cl | $CH_3$ | $CH_2-CF_3$ | $CH_3$ | |
| 8 | 2-Cl | $C_2H_5$ | $CH_2-CF_3$ | $CH_3$ | |
| 9 | $3-NO_2$ | $CH(CH_3)_2$ | $n-PrO-CH_2CH_2$ | $CH_3$ | |
| 10 | $3-NO_2$ | $CH_3$ | $C_6H_5CH_2N(CH_3)CH_2CH_2$ | $CH_3$ | Nicardipin |
| 11 | $2,3-Cl_2$ | $C_2H_5$ | $CH_3$ | $CH_3$ | Felodipin |
| 12 | 2,3=N-O-N= | $C_2H_5$ | $C_2H_5$ | $CH_3$ | |
| 13 | 2,3=N-O-N= | $CH_3$ | $CH(CH_3)_2$ | $CH_3$ | |
| 14 | $3-NO_2$ | $C_2H_5$ | $C_2H_5$ | $CH_2OH$ | |
| 15 | $3-NO_2$ | $CH_3$ | $CH_3$ | CN | |

n-Pr = n-Propyl

Insbesondere seien genannt: Nifedipin, Nimodipin, Nitrendipin und Nisoldipin.

Als Lipide der Liposomenmembran kommen in Betracht:

Phosphatidylcholin (Lecithin), Phosphatidylserin, Phosphatidylethanolamin, Phosphatidylglycerol, Phosphatidylinositol, Sphingomyelin, Cholesterin.

Das wäßrige Medium weist bevorzugt einen pH-Wert von 7,0 bis 7,5 auf. Es kann übliche Hilfs- und Zuschlagstoff wie beispielsweise physiologische Kochsalzlösung oder andere isotone Salzlösungen enthalten.

Darüber hinaus kann die Lösung zum Zwecke der pH-Einstellung übliche pharmazeutisch unbedenkliche Puffer wie Phosphatpuffer enthalten.

Das Gewichtsverhältnis von Lipiden (welche in Wasser die Liposomen bilden) zu Wasser beträgt bervorzugt 20 bis 100 Gewichtsteile Wasser pro ein Gewichtsteil Lipid.

Bei längerer Lagerung der erfindungsgemäßen Arzneizubereitung sowie bei tiefer Temperatur können unter Umständen die Dihydropyridine die Liposomenmembran verlassen und im wäßrigen Außenmedium auskristallisieren.

Um die Erscheinung weitgehend zu verhindern, können, bezogen auf die wäßrige Lösung, bis zu 20 Gew.-%, bevorzugt bis zu 10 Gew.-%, eines Polyalkohols zugesetzt werden.

Als Polyalkohol seien bevorzugt solche genannt, die 2-6 C-Atome und 2-6 Hydroxylgruppen aufweisen, sowie Mono-, Oligo- und Polysaccharide.

Besonders bevorzugt seien genannt: Dihydroxypropan, Glycerin, Mannit, Glucose, Sucrose und Dextran.

Die erfindungsgemäße Arzneizubereitung kann somit unbedenklich eingefroren werden.

Auf diese Weise erhöht sich die Lagerfähigkeit beträchtlich.

Ein weiterer Gegenstand der Erfindung ist ein Verfahren zur Herstellung der Arzneizubereitungen, das dadurch gekennzeichnet ist, daß man 1 Gewichtsteil des Dihydropyridins und 5-500 Gewichtsteile Lipid in organischen Lösungsmitteln löst, anschließend das Lösungsmittel entfernt und den Rückstand nach Zugabe eines wäßrigen Mediums mit einem pH-Wert von 6,5 bis 8 bei Temperaturen zwischen 20 und 80°C dispergiert.

Als organische Lösungsmittel kommen beispielsweise in Betracht: Methanol, Ethanol, Aceton oder leicht flüchtige Halogenkohlenwasserstoffe.

Die Dispersion wird unter Anwendung bekannter Methoden, bevorzugt mittels Ultraschall, mittels Schnellrührer oder mittels eines Hochdruckhomogenisators hergestellt. Die Dispersion kann nach ihrer Herstellung thermisch sterilisiert werden. Ebenfalls ist es möglich, diese in ein

trockenes Pulver durch Gefriertrocknung zu überführen. Das so getrocknete Pulver kann anschließend bei Bedarf mittels wäßriger Lösungen vom pH 3,0-8,5 wieder redispergiert werden.

Die Erfindung sei anhand folgender Beispiele verdeutlicht:

Beispiel 1

Nimodipin-Liposomen werden mit der Beladungskonzentration 0.3 mg/ml oder 1 mg/ml durch Ultrabeschallung hergestellt. Für 10 ml-Ansätze werden 3 bzw. 10 mg Nimodipin und 200 mg Sojabohnen-Lecithin in ca. 0,2 ml Methanol/2 ml Chloroform gelöst. Das Lösungsmittel wird abgedampft, bis ein fester, trockener Film zurückbleibt. Nach Zugabe von 10 ml 0,02 M Na-Phosphatpuffer (pH 7.3) wird mit einem Eintauch-Ultraschallgerät (Branson B 12 mit Standard 1/2 Zoll-Finger) 8 min lang bei etwa 55°C unter Argon-Schutzgas dispergiert. Anschließend wird die Dispersion bei 1200 g 10 Min lang zur Abtrennung des Titanpartikelabriebs vom Ultraschallfinger zentrifugiert. Alle Präparationen werden wegen der Lichtempfindlichkeit von Nimodipin bei Na-Licht ausgeführt. Zur Bestimmung der mittleren Teilchengröße der Liposomen ist die Methode der dynamischen Lichtstreuung geeignet. Die Nimodipin-Liposomen weisen eine mittleren Durchmesser im Bereich zwischen 50 und 100 nm auf.

Beispiel 2

Jeweils 3 bzw. 10 mg Nimodipin und 200 mg Sojabohnen-Lecithin werden in 0,2 ml Methanol/2 ml Chloroform gelöst. Das Lösungsmittel wird abgedampft, bis ein fester, trockener Film zurückbleibt. Dem für die Herstellung der Liposomen erforderlichen wäßrigen Medium werden vor der Dispergierung 10 Gew.-% Mannit zur Stabilisierung der Nimodipin-Beladung der Liposomen zugesetzt. Die weitere Herstellung, Aufarbeitung und analytische Größenbestimmung der Liposomen erfolgt wie in Beispiel 1.

Beispiel 3

Wie Beispiel 2. Dem wäßrigen Medium werden jedoch vor der Dispergierung 5.2 Gew.-% Glycerin zur Stabilisierung der Nimodipin-Beladung der Liposomen zugesetzt. Die mit Glycerin-Zusatz hergestellten Liposomen werden im Gefrierschrank bei -20°C langsam eingefroren. Nach dem Auftauen der Dispersionen sehen die Liposomenpräparationen gleich aus wie vor dem Einfrieren. Die Messungen mit Hilfe der dynamischen Lichtstreuung ergaben die gleichen mittleren Liposomen-Teilchengrößen wie vor dem Einfrieren.

Beispiel 4

20 mg Nimodipin und 2 g Sojabohnenlecithin werden in 2 ml Methanol/20 ml Chloroform gelöst. Das Lösungsmittel wird abgedampft. Der Feststoffrückstand wird als Pulver einem Volumen von 100 ml von wäßrigem Na-Phosphatpuffer (20 mM), enthaltend 4 % Propandiol, zugegeben. Der Feststoff wird mit einem Schnellrührer ca. 60 Minuten lang dispergiert. Es entsteht eine Nimodipin-haltige Liposomen-Suspension.

Beispiel 5

Dem Phosphatidylcholin (Lecithin) (200 mg) wird Phosphatidylserin (2 mg) als negativer Ladungsträger zugegeben. Der weitere Präparationsgang erfolgt wie in Beispiel 1 beschrieben.

Beispiel 6

Als Phospholipid wird statt 200 mg Sojabohnenlecithin die Mischung von 200 mg Sphingomyelin plus 2 mg Phosphatidylserin verwendet. Die weitere Präparation erfolgt wie in Beispiel 1. Der gemessene mittlere Durchmesser der Liposomen beträgt 58 nm.

Beispiel 7

Als Phospholipide werden Dipalmitoylphosphatidylcholin (200 mg) und Phosphatidylserin (2 mg) verwendet. Die weitere Aufarbeitung erfolgt wie in Beispiel 1. Der mittlere Durchmesser der Liposomen beträgt 51 nm.

Beispiel 8

Die Chloroformlösung von 100 mg Eilecithin, 8 mg Dicetylphosphat und 5 mg Nifedipin wird unter Argonbegasung abgedampft. Nach Zusatz von 10 ml 0,1 M Na-Phosphatpuffer pH 7.3 wird 10 min beschallt bei 40-45°C und Na-Licht.

Beispiele 9-11

3 mg der Dihydropyridinverbindung (s. Tabelle 1) und 200 mg Sojabohnenlecithin werden in 0,2 ml Methanol/2ml Chloroform gelöst. Das Lösungsmittel wird unter Argon-Begasung abgedampft. Nach Zugabe von 0,02 M Phosphatpuffer pH 7,3, der 5,2 % Glycerin enthält, wird mit Ultraschall bei 55°C und Argon dispergiert. Die Dispersionen werden bei 1200 g 10 min zentrifugiert. Alle Präparationen

werden wegen der Lichtempfindlichkeit der Dihydropyridin-Derivate bei Natrium-Licht ausgeführt. Die mittlere Teilchengröße der Liposomen wird mit der Methode der dynamischen Lichtstreuung bestimmt. Der gemessene mittlere Durchmesser der Liposomen, die mit den als Beispiele in Tabelle 1 aufgeführten Dihydropyridin-Derivaten beladen sind, ist in der Tabelle angegeben.

Tabelle 1

Mittlere Teilchengröße und Durchmesserverteilungsbreite der DHP-beladenen Liposomen

| Beispiel | Formel | d /nm/ | K2 |
|---|---|---|---|
| 9 | 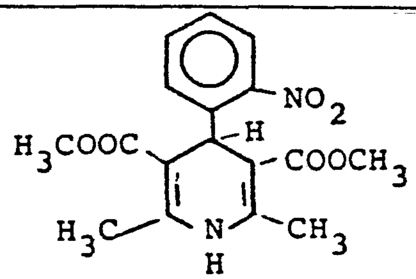 | 57 | 0,28 |
| 10 | 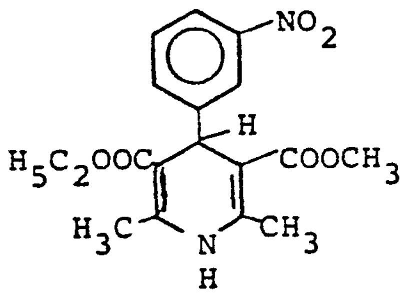 | 74 | 0,45 |
| 11 | 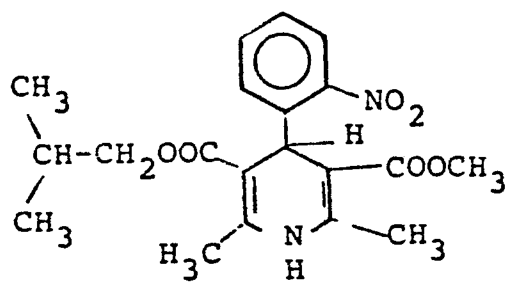 | 81 | 0,42 |

DHP = Dihydropyridin-Derivat

d = mittlere Teilchengröße der Liposomen

K2 = Durchmesserverteilungsbreite

Beispiel 12

28 mg Nimodipin (s. Tabelle Seite 5) und 2,1 g Eilecithin werden in 5 ml Methanol/15 ml Chloroform gelöst. Das Lösungsmittel wird unter Argon-Begasung abgedampft. Nach Zugabe von 70 ml 0,02 M Phosphatpuffer pH 7,3, der 8 % Trehalose enthält, wird mit einem Eintauch-Ultraschallgerät bei 55°C unter Argon-Begasung dispergiert. Nach der Zentrifugation der Dispersion bei 1200 g für 10 Minuten weisen die Nimodipin-Liposomen einen mittleren Durchmesser von 110 nm auf.

Die Dispersion wird anschließend in 10 ml Portionen in Glasröhrchen aufgeteilt. Die Glasröhrchen werden mit Spezialstopfen fest verschlossen und 20 Minuten bei 120°C sterilisiert. Wie die Messungen mit der Methode der dynamischen Lichtstreuung zeigten, ist die mittlere Teilchengröße unverändert gegenüber den Werten vor der thermischen Behandlung.

Beispiel 13

Die Präparation erfolgt wie in Beispiel 1 mit dem Unterschied, daß die thermische Behandlung in 2 Schritten bei der Temperatur von 70°C je eine Stunde lang durchgeführt wird. Zwischen den beiden thermischen Schritten von 70°C werden die Röhrchen 24 Stunden bei Raumtemperatur aufbewahrt. Der mittlere Teilchendurchmesser ist vor und nach der thermischen Behandlung identisch.

Beispiel 14

Die Präparation erfolgt wie in Beispiel 1 mit dem Unterschied, daß nach der thermischen Behandlung die Dispersion in einem konventionellen Gefriertrocknungsgerät lyophilisiert wird. Durch die Lyophilisation erhält man ein trockenes Pulver. Werden zu 1 g des mehrere Wochen bei Raumtemperatur aufbewahrten Trockenpulvers 30 ml destilliertes Wasser hinzugegeben, entsteht durch Schütteln mit der Hand in wenigen Minuten eine homogene wäßrige Dispersion. Der mittlere Teilchendurchmesser dieser Dispersion ist unverändert gegenüber dem vor der Lyophilisation erhaltenen Meßwert (110 nm).

Beispiel 15

150 g Eilecithin sowie 1 g Nimodipin werden in 200 ml Ethanol gelöst. Das Lösungsmittel wird bei 50-60°C abgedampft. Der so präparierten Trockensubstanz werden 5 l eines wäßrigen Zitratpuffers (pH = 6,7) zugesetzt. Der Puffer enthält 4,7 % D-Mannit. Nach einer ca. 24-stündigen Inkubationszeit wird das Gefäß mit dem Feststoff und der wäßrigen Phase gut geschüttelt, wobei eine Rohdispersion entsteht. Diese Rohdispersion wird in einem Hochdruckhomogenisator weiter dispergiert. Das verwendete Gerät (Strahl-Dispergator) arbeitet bei einem Druck von 250 bar. Es ermöglicht einen Kreislaufbetrieb (Rückführung des Auslaufs in den Einlaufkanal). Nach 20 Durchläufen wird eine Liposomen-Dispersion erhalten, deren mittlere Teilchengröße 163 nm beträgt.

Beispiel 16

Pharmakologische Wirkung von Nimodipin-Liposomen

Die therapeutische Anwendung der parenteralen Applikationsform von Nimodipin beruht auf seiner erweiternden Wirkung auf die Gehirngefäße. Seine derartige Wirkung - eine Erhöhung der Blutversorgung des Gehirns - läßt sich im Tierexperiment mit Hilfe der $^{133}$Xenon-Clearance im Gehirn nachweisen.

An Katzen, die mit Ketamin (R) narkotisiert sind, wird das Auswaschen (clearance) von dem intraarteriell injizierten $^{133}$Xenon als Maß für die Gehirndurchblutung verwendet. Das radioaktiv markierte $^{133}$Xenon wird in die ins Gehirn führende Arterie injiziert und die radioaktive Emission über dem Schädeldach (über dem temporooccipitalen Cortex) mit Hilfe von $\gamma$-sensitiven Aufnehmern registriert. Aus der so gewonnenen Auswaschkurve wird dann die Größe der Durchblutung der grauen Gehirnsubstanz nach der Formel

$$f = \frac{\lambda \ln 2}{T_{1/2}}$$ ermittelt.

$\lambda$ = Verteilungskoeffizient von Xenon

$T_{1/2}$ = Halbwertzeit des initialen, linearen Teils der Clearance-Kurve

Gleichzeitig wird der arterielle Mitteldruck in der Aorta femoralis mittels eines Druckaufnehmers Statham registriert.

Nimodipin in der Liposomenformulierung wurde in steigenden Dosen intraarteriell in die ins Gehirn führende Arterie appliziert. In dieser Form steigert Nimodipin die Gehirndurchblutung in einem Dosenbereich von 0.0001 - 0.00315 dosenabhängig (Tab. 2).

Tabelle 2

Erhöhung der Gehirndurchblutung an Katzen mit Nimodipin in Liposomen

| Nimodipin mg/kg intraarteriell | 0.0001 | 0.0003 | 0.001 | 0,003 |
|---|---|---|---|---|
| Erhöhung der Gehirndurchblutung in % | 18±5.1 | 34±8.1 | 37±7.5 | 57±16,0 |

Die maximale Erhöhung der Gehirndurchblutung nach Nimodipin in der Liposomen-Formulierung wurde in der Dosis von 0.003 mg/kg erreicht. Sie betrug 57 ± 16 % gegenüber dem Ausgangswert.

Patentansprüche

1. Parenteral applizierbare Arzneizubereitung, welche Liposomen mit einem mittleren Durchmesser von 20-1000 nm enthält, wobei die Liposomenmembran folgende Bestandteile aufweist:

   a) 1 Gewichtsteile eines Dihydropyridins
   b) 5-500 Gewichtsteile Lipide

   und wobei die Liposomen in einem wäßrigen Medium verteilt sind, welches einen pH-Wert von 3,0-8,5 aufweist.

2. Arzneimittelzubereitung gemäß Anspruch 1 enthaltend Liposomen mit einem mittleren Durchmesser von 50-200 nm.

3. Arzneimittelzubereitung gemäß Anspruch 1 enthaltend Liposomen, deren Membran 20-200 Gewichtsteile Lipid enthält.

4. Arzneimittelzubereitung gemäß Anspruch 1, dessen wäßrige Lösung einen pH-Wert von 7 bis 7,5 aufweist.

5. Arzneimittelzubereitung gemäß Anspruch 1 enthaltend zusätzlich bis 20 Gew.-% eines Polyalkohols.

6. Arzneimittelzubereitung gemäß Anspruch 1 enthaltend als Dihydropyridin eine Verbindung der Formel I

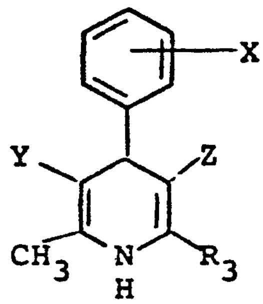

I

in der

Y die Gruppe $-CO_2R_1$,

$R_1$ $C_1$-$C_4$-Alkyl, gegebenenfalls substituiert durch $C_1$-$C_3$-Alkoxy,

Z die Gruppen $-COOR_2$,

$R_2$ $C_1$-$C_{10}$-Alkyl, gegebenenfalls substituiert durch $C_1$-$C_3$-Alkoxy, Trifluormethyl, N-Methyl-N-benzylamino, Benzyl,

$R_3$ $C_1$-$C_4$-Alkyl, Cyano, Hydroxymethyl und

X 2- bzw. 3-Nitro, 2,3-Dichlor, 2,3-Ringglied bestehend aus =N-O-N=, 2- bzw. 3-Trifluormethyl

bedeuten.

7. Verfahren zur Herstellung von parenteral applizierbarer Arzneizubereitung, welche Liposomen mit einem mittleren Durchmesser von 20-1000 nm enthält, wobei die Liposomenmembran folgende Bestandteile aufweist:

   a) 1 Gewichtsteil eines Dihydropyridins
   b) 5-500 Gewichtsteile Lipide

   und wobei die Liposomen in einem wäßrigen Medium verteilt sind, welches einen pH-Wert von 3,0 - 8,5 aufweist, dadurch gekennzeichnet, daß man 1 Gewichtsteil des Dihydropyridins und 5-500 Gewichtsteile Lipid in organischen Lösungsmitteln löst, anschließend das Lösungsmittel entfernt und den Rückstand nach Zugabe eines wäßrigen Mediums mit einem pH-Wert von 3,0 bis 8,5 bei Temperaturen zwischen 20 und 80°C dispergiert.

8. Verfahren gemäß Anspruch 7, dadurch gekennzeichnet, daß man als organische Lösungsmittel Methanol, Ethanol, Aceton oder leicht flüssige Halogenkohlenwasserstoffe verwendet.

9. Verfahren gemäß Anspruch 7, dadurch gekennzeichnet, daß der Rückstand nach Entfernung des organischen Lösungsmittels mittels Ultraschall, Schnellrührer oder Hochdruckhomogenisator dispergiert wird.

10. Verfahren gemäß Ansprüchen 8-9, gekennzeichnet dadurch, daß die Liposomendispersion durch mindestens 20-minütiges Erhitzen auf 120°C sterilisiert wird.

11. Verfahren gemäß Ansprüchen 8-10, gekennzeichnet dadurch, daß die Liposomendispersion im Abstand von mindestens 24 h jeweils 1 Stunde lang auf 70°C erhitzt wird.

12. Verfahren gemäß Ansprüchen 9-11, dadurch gekennzeichnet, daß die Liposomen-Dispersion nach Zusatz eines Zuckers oder eines Polyalkohols mit bekannten Geräten lyophilisiert wird, wodurch ein stabiles Pulver entsteht, das unbegrenzt lagerungsfähig ist und jederzeit durch Zusatz von Wasser redispergiert werden kann.